# EUROPEAN PATENT APPLICATION

(11) **EP 1 582 221 A1**
(43) Date of publication of application: **05.10.2005**
(21) Application number: 04290848.3
(22) Date of filing: 31.03.2004
(51) Int. Cl.: A61K 47/10, A61K 47/12, A61K 47/26, A61K 47/36, A61K 47/38, A61K 47/40

(54) **Pharmaceutical oral compositions with a taste masking effect**

(71) Applicant: CHIESI S.A., 92400 Courbevoie (FR)
(72) Inventor: Plouvier, Thierry, 78150 le Chesnay (FR); Kilhoffer, Daniel, 27000 Evreux (FR); Le Peillet-Feuillet, Eliane, 92100 Boulogne Billancourt (FR); Tubery, Françoise, 91430 Igny (FR)
(74) Representative: Vaillant, Jeanne

(57) **Abstract**

The present invention relates to compositions comprising a) at least a pharmaceutically active substance which has an unpleasant taste; and b) at least a non-lipid taste masking association comprising at least an acid and at least a binder, and c) at least a filler. It also relates to oral pharmaceuticals comprising these compositions and processes for making and administering such compositions.

## Description

### Field of the invention

This invention relates to freeze dried oral compositions, having improved palatability, comprising a pharmaceutically active substance with an unpleasant taste. It also concerns solid, oral pharmaceutical compositions and dosage units. It also relates to processes for preparing such compositions.

### Background

Dosage forms which are deemed to rapidly disintegrate on being placed in the mouth of the patient, or by sucking or crunching, thereby delivering the desired dose of the pharmaceutically active substance are particularly useful when no additional water or beverage can be used for practical or medical reasons, for example when traveling or if any beverage in-take should be avoided.

Dosage units occupy an important position amongst various dosage forms, not only for human adults, but also for children, old people, and even for animals. Examples include people or animals having difficulties related to or being uncooperative in swallowing and/or taking tablets or capsules.

These dosage forms can be prepared by usual multistep processes. Amongst them, some processes comprise a lyophilization step during which the solvent is removed from a mixture comprising said solvent, the pharmaceutically active substance and a carrier material.

Although the solid dosage forms as described above overcome the problem of swallowing tablets or capsules, the patient will taste the pharmaceutically active substrate as the dosage form disintegrates. For some pharmaceutically active substances, the taste, if slightly unpleasant, can be rendered acceptable by the use of sweetening agents or flavoring agents which modulate the taste. However, for some pharmaceutically active substances, an unpalatable product will still be produced, despite the use of sweetening agents and flavoring agents. Unpalatable products decrease a patient's compliance. It is known to prepare particles by a method comprising extrusion of a pasty mixture comprising the active substance, then freeze-drying the moist particles. WO 94/21371 discloses such a method and teaches to coat the active substance in order to avoid its taste, when a non palatable substance is to be processed.

This means however that the process comprises a supplemental step of coating within the process and, apart from the economical drawbacks of such a coating step, the coating may adversely modulate the bioavailability of the active substance.

It is also known to convert the active substance into another form with less unpleasant taste (WO96/13252 Yearwood). Some active substances are however less bioavailable in this other form.

It is also known to associate the active substance with a lipid. Some active substances are however not easily compatible with lipids. The compositions disclosed in WO98/35656 (Grother) involve the use of a lipid/pharmaceutical active combination. However, lipids such as phospholipids, lecithins, glycolipids are subject to oxidation and should be protected from oxidants and light, (Handbook of Pharmaceutical excipients, R.C. Rowe, P. Sheskey, P.J. Weller, fourth edition, Pharmaceutical press) and, therefore, associations containing them usually present a diminished stability when stored under normal conditions and should be kept in well closed containers.

There is therefore still a need for palatable dosage units and freeze dried particles for preparing the same in which the active substance remains at least as bioavailable as in other forms, and is easy to produce and to process into a galenic form. For example, there is a need to compress an active substance into a solid galenic form, such as tablets, without stability problems and without coating the active substance.

For example, Piroxicam is a compound belonging to the class of the Non Steroidal Anti-Inflammatory drugs (NSAI) which, due to its remarkable analgesic and antiphlogistic activity, is employed in the treatment of arthro-rheumatic diseases. Furthermore, piroxicam is responsible for lesion effects on the gastrointestinal mucosa.

Piroxicam is however practically insoluble in water, and this may represent a limiting factor for an optimal employment of the substance. Piroxicam is therefore advantageously complexed by inclusion into α-, p- or γ-type cyclodextrins. The obtained complex possesses a high solubility, is rapidly absorbed and is better tolerated. This complex is described in detail in EP 84113923.1, which is hereby expressly incorporated by reference in its entirety.

However, the complex has the drawback in that it retains the unpleasant bitter taste of piroxicam, which prevents it from being easily incorporated into oral compositions or dosage units which are deemed to disintegrate in the mouth.

Accordingly, there is a need in the art to formulate particles and dosage units without a bitter unpleasant taste and in which the piroxicam or the complexed piroxicam is as bio-available as in other forms, and can be easily processed, the formulation not necessarily including the use of flavoring agents or a further coating step. According to the invention, unpalatable substances can thus be easily processed and subsequently formulated without the need for flavoring agents or a coating step.

### SUMMARY OF THE INVENTION

The present invention relates to compositions having improved palatability prepared by freeze drying a mixture comprising a) at least one pharmaceutically active substance having an unpleasant taste; and b) at least one non-lipidic taste masking association comprising: b1) at least one acid and b2) at least one non-lipidic binder in sufficient amount to improve the palatability of the active substance having an unpleasant taste, and c) at least one filler. Preferably, these compositions are prepared into oral pharmaceuticals.

Suitable acids can include citric acid or fumaric acid. The non-lipidic binder can be selected from the group consisting of pectin, gelatin, xanthan gum, guar gum, cellulose derivatives, soy derivatives, polysaccharides, pullolan, povidone and derivatives, polyethylene glycol, chitosan and its derivatives, fermentation obtained sugars like xanthan, alginates, and their mixtures, for example.

Advantageous binders can be selected from the group of pectin, gelatin, xanthan gum, and apple pectin, and their mixtures.

Suitable filler can be selected from the group consisting of dextrose, lactose, saccharose, fructose, galactose, starch, cellulose and its derivatives, polyols like mannitol, xylitol, sorbitol, maltodextrin, amino acids, and their mixtures. In advantageous embodiments, the filler is dextrose.

Suitable pharmaceutically active substances can include piroxicam, piroxicam-β-(cyclodextrine) (BCDP), ambroxol hydrochloride, paracetamol, domperidone, nimesulide, ibuprofen, morniflumate, apomorphine and dihydroergocryptine.

In specific embodiments, the pharmaceutically active substance can be non-complexed piroxicam or paracetamol. In other embodiments, the pharmaceutically active substance is complexed with a cyclodextrin.

In further embodiments, the compositions herein can comprise at least 5% acid by weight, at least 6% binder by weight, and at least 20% filler by weight.

In a preferred embodiment, the active substance is complexed piroxicam, and the weight ratio between the acid and binder is equal to or less than 1. In additional embodiments, the active substance is BCDP, and the percentage of acid, based on total weight, is between 5 and 10%, the percentage of the binder, based on total weight, is between 6 and 10% and the percentage of the filler, based on total weight, is between 20 and 30%.

In other aspects, the compositions are freeze-dried aqueous or hydroalcoholic gels. Freeze-dried aqueous or hydroalcoholic gels can include free-flowing compressible powders, for example.

Further embodiments include sieved freeze dried particles comprising a) at least a pharmaceutically active substance which has an unacceptable taste; b) at least a non-lipidic taste masking association comprising: b1) at least an acid and b2) at least a non-lipidic binder; and c) at least a filler.

Further embodiments include free-flowing compressible powder-like freeze-dried particles comprising a) at least a pharmaceutically active substance which has an unacceptable taste; b) at least a non-lipidic taste masking association comprising: b1) at least an acid and b2) at least a non-lipidic binder; and c) at least a filler.

Further embodiments include methods for the preparation of freeze dried particles including a pharmaceutically active substance having an unpleasant taste, and a non-lipidic taste masking association comprising an acid and a binder, and a filler. The methods herein can include the following steps: preparing an aqueous or hydroalcoholic solution or suspension of the active substance having an unpleasant taste, preparing an aqueous or hydroalcoholic gel comprising said aqueous or hydroalcoholic solution or suspension and a non-lipidic binder, an acid, and a filler, homogenizing the aqueous or hydroalcoholic gel, freeze drying said homogenized gel and, recovering the freeze dried material. The recovered freeze dried material can then be made into an oral pharmaceutical composition. Suitable oral pharmaceutical dose units can be sachet form or tablets.

In certain aspects, it can be advantageous to sieve the freeze-dried compositions then recover the resulting sieved product. In preferred embodiments, the freeze-dried material is sieved through a mesh of between about 200 µm to 5 mm.

It can also be advantageous to provide the acid, the binder, and filler successively to the solution or suspension, either simultaneously or extemporaneously.

In one aspect, the advantage of the invention process is the fact that the filler is added to the above mixture prior to the lyophilization step, leading to a simple production process for the freeze dried particles.

Preferred pharmaceutically active substances include BCDP, for example.

Further embodiments, include processes for administering an oral pharmaceutical composition having improved palatability. Such processes can include the following steps: providing an oral composition having a pharmaceutically active substance with an unpleasant taste, an acid and a non-lipidic binder in sufficient amount to improve the palatability of the active substance having an unpleasant taste, and administering said oral pharmaceutical composition to a patient in need, wherein said composition has improved palatability.

Preferred pharmaceutically active substances can include piroxicam or piroxicam/cyclodextrin complex. Preferred binder include sugars, and preferred acids include citric and fumaric acid.

Advantageous compositions for administration, including oral pharmaceuticals such as tablets, can also include a filler in which case the filler is mixed with the active substance, the acid and the non lipidic binder prior to the freeze-drying step. This is not exclusive of other excipients, like other filler which might be used within the formulation of the pharmaceutical composition, as it appears here below.

In other aspects, the invention can include pharmaceutical compositions having improved palatability, comprising piroxicam or piroxicam/cyclodextrin complex, a non-lipidic binder, and an organic acid, and a filler wherein said composition results from lyophilization of a homogeneous liquid or gel. As above explained the compositions can further include a further filler for processing.

In some embodiments, the compositions described herein, can be in the form of a particulate material. The compositions can also be pharmaceutical tablets.

In further embodiments, the organic acid can have from 2 to 8 carbon atoms and one or more carboxylic groups.

In additional embodiments, the non-lipidic binder can be a thickening or gelling agent. In more specific embodiments, the non-lipidic binder can be a polypeptide, a polyhydroxy compound, or a carbohydrate.

Additional embodiments include methods for making a pharmaceutical material having improved palatability, comprising preparing a homogeneous liquid or gel comprising piroxicam or piroxicam/cyclodextrin complex, a non-lipidic binder, an organic acid and a filler; and lyophilizing the liquid or gel. In further aspects, the methods herein can include sieving said lyophilized liquid or gel. In more specific embodiments, the methods can also include a step for formulating an oral pharmaceutical composition from the lyophilized gel or liquid.

In certain aspects, the acid and the binder can be premixed with water prior to being combined with said piroxicam or piroxicam/cyclodextrin. Additional aspects can include adding a filler to the acid binder, and water premix.

The compositions and methods herein can include both effervescent and non-effervescent tablets.

### DETAILED DESCRIPTION

The present invention relates to oral compositions having improved palatability comprising unpleasant tasting pharmaceutically active substance(s), including, but not limited to, piroxicam or to beta cyclodextrin piroxicam (BCDP).

According to one aspect of the invention, sievable freeze dried particles comprising a) at least a pharmaceutically active substance which has an unpleasant taste; b) at least a non lipidic taste masking association comprising: b1) at least an acid and b2) at least a non lipidic binder; and c) at least a filler is provided.

The use of a non lipidic masking association according to the invention, preferably the association of citric acid and/or fumaric acid, with a binder, preferably chosen from pectin and/or gelatin and/or xanthan gum allows to mask unpalatable taste. Furthermore the use of a filler allows to easily process the obtained freeze dried material.

The Applicant has shown that the acid alone can modify the bitterness because of its acidity, but the taste can remain unpalatable. Furthermore, the use of a binder and/or a filler, without the acid does not generally mask the bitterness. It has also been shown that the use of a binder alone, without filler, does not generally lead to processed particles. This has been shown in the comparative examples.

According to the invention, the taste masking effect does not depend on the use of any further sweetening or flavoring agent.

Compared to the use of lipids, the compositions of the present invention, with a non lipidic taste masking association present a better stability.

According to another aspect, a process for the preparation of freeze dried particles comprising a pharmaceutically active substance which has an unpleasant taste, a non lipidic taste masking association comprising an acid and a non lipidic binder, and a filler is provided, comprising at least the following steps:
a) preparing an aqueous solution or suspension of the active substance or an hydroalcoholic solution or suspension of the active substance;
b) obtaining an aqueous or hydroalcoholic gel comprising said aqueous solution or suspension or said hydroalcoholic solution or suspension and at least a non-lipidic binder, at least an acid and at least a filler;
c) eventually homogenizing said aqueous or hydroalcoholic gel obtained in step b);
d) freeze drying said homogenized gel; and
e) recovering the freeze dried material.

In certain aspects, the processes of the invention do not require a coating step and are easy to carry out.

In further aspects, the freeze-drying process can be carried out with a gel, which does not need to be extruded, because such a gel is a non-pasty material. The freeze dried material obtained can then be easily sieved.

The process can further comprise a step of sieving the freeze dried material to obtain sieved freeze dried particles.

In one aspect of the invention the particles are free-flowing compressible powder-like freeze-dried particles.

The minimal size of the sieved particles can advantageously be about 200-5000 µm, preferably 250-400 µm, and more preferably about 315 µm, particularly in case of a further step of preparing tablets.

Furthermore, the obtained particles might be directly further processed. A practitioner can easily process the sieved freeze dried particles, which can be directly and easily compressed to obtain oral tablets without bitter or unpalatable taste.

According to a third aspect, pharmaceutical oral compositions comprising freeze dried particles as defined above or obtained by the above process are provided. These particles can be used in sachet form for example, or directly compressed to obtain tablets. Such tablets can be crunched and/or sucked by a patient or animal. These tablets disintegrate and the result of their disintegration can be swallowed without further liquid.

The oral pharmaceutical formulations may also be in the form of a sachet, and/or a pulverulent material to be swallowed with a liquid like water to be extemporaneously mixed with the particles, just before in-take.

The term "active substance," according to the invention, relates to a pharmaceutically substance, which in form has an unpleasant taste.

Classes of pharmaceutically active substances which may be formulated in the process of the present invention include non steroidal anti-inflammatory drugs, antacids, analgesics, antalgics, sedatives, antipyretics, antispasmodics, coronary vasodilatators, peripheral vasodilatators, cerebral vasodilatators, anti-injectives, antibiotics, antiparasitics, antineoplasics, anxiolytics, tranquilizers, CNS stimulating agents, antihistaminics, laxatives, drugs for digestive motricity, anticoagulant, drug for use in thyroid dysfunction, nutriments, nutritional adjuvants, immuno-depressives, cholesterol-lowering agents, hormones, sex hormones, enzymes, antianginal drugs, anti-migraine drugs, myorelaxants, hypoglycemics, anorexiants, expectorants, antitussives, decongestants, antinauseants, hematopoietic drugs, uricosurics, plant extracts, radio contrast media, anti-anginals, anti-anxiety drugs, anti-arrhythmics, anti-bacterials, anti-diarrhoeals, anti-depressants, antiepileptics, anti-fungals, anti-hypertensives, anti-inflammatory agents, anti-virals, cardiac agents, contraceptives, cough suppressants, cytotoxics, decongestants, diuretics, drugs for genito-urinary disorders, drugs for asthma disorders, drugs for use in parkinsonism and related disorders, drugs for use in rheumatic disorders, hypnotics, minerals, vitamins, lipid lowering drugs and the like.

Amongst these substances, particularly, Non-Steroidal Anti-Inflammatory (NSAI) drugs may be processed according to the process of the invention or comprised in the freeze dried particles or pharmaceutical compositions of the invention.

Specific examples of active substances that can be used with the teachings herein include, paracetamol and dihydroergocryptine.

Preferably, the active substance is piroxicam. This substance may be used in its complexed form, namely the forms where piroxicam is complexed by inclusion into α-, β- or γ-type cyclodextrins.

The cyclodextrins are natural cyclic compounds consisting of 6(α), 7(β) or 8(γ) (1 → 4) D-glucopyranosidic units. In this complex, piroxicam and the cyclodextrins may be present in ratios comprised between 1:1 and 1:10, preferably 1:2.5.

However, non-complexed piroxicam may also be used.

"Non lipidic taste masking association" according to the invention comprises an acid and a non lipidic binder.

Particularly, citric acid, but also fumaric acid is convenient as the acidic constituent of the non-lipidic association.

In further embodiments, the acid can be an organic acid. In more specific embodiments, the acid can contain 2 to 8 carbon atoms and one or more carboxylic groups. In specific embodiments, the organic acid is selected from the group consisting of citric, lactic, fumaric, sorbic, malic, and propionic acid. The acid is preferably chosen in such a way that, in combination with the binder, it does not prevent the formation of a gel.

The acid can be used in an amount of about 5% to about 50% by weight of dried material. The acid can also be used in between about 5% to 10%, or 5% to 16% by weight of dried material.

The term "binder," according to the invention, relates to the following groups of compounds, and the like: pectin, gelatin, xanthan gum, guar gum, cellulose derivatives, soya derivatives, polysaccharides, pullolan, povidone and derivatives, polyethylene glycol, chitosan and its derivatives, fermentation obtained sugars like xanthan, alginates, and their mixtures. Cellulose derivatives can include ethylcellulose, hydroxyethylcellulose, hydroxypropylcellulose, carboxymethylcellulose, sodium hydroxypropylmethylcellulose, and their mixtures, for example.

Other substances which may be used as the binder are, for example, hydrolyzed dextrose, dextran, dextrin, maltodextrin, corn-syrup solids, carrageenan, agar, locust bean gum, tragacanth, conjac flower, rice flower, wheat gluten, sodium starch glycolate, soy fiber protein, potato protein, papain, glycine, mannitol, carbomer, acacia, casein, povidone and derivatives and their mixtures.

The preferred binders are of vegetal origin like pectin and xanthan gum, and the particularly preferred is pectin. Gelatin is also a preferred binder.

The binder can be used in an amount of about 6% to 30% by weight of the obtained dried material. The upper limit however depends on the binder. Other ranges include about 6% to 10%, and about 6% to 16%, for example.

Preferably, the upper limit of pectin can be of about 7% in the case of piroxicam complexed with betacyclodextrin, hereinafter BCDP (betacyclodextrin piroxicam) and about 15% in case of non complexed piroxicam.

Pectin can be used as a stabilizer of the formed gel. In certain aspects, the gel allows for easily processed particles, after the freeze-drying step and the sieving step.

Pectin, or alginates, are polysaccharides with D-galacturonic acid as the major constituent. Association thereof with an acid such as citric or fumaric acid can induce a modification of ionization of galacturonate residues, influencing the structure of gel.

Different types of interactions may occur depending on the structure of the active substance. Possible direct interactions include hydrogen bonding between carbohydrates, ionic interaction with galacturonate residues. Indirect interaction is related to the surface active properties of the hydrocolloid agent acting like micelles.

In a preferred embodiment, the active substance is complexed piroxicam and the weight ratio of citric acid/binder is equal or less than 1, and more preferably the acid is citric acid and the binder is apple pectin.

The term "filler," according to the invention, relates to a diluent selected from dextrose, lactose, saccharose, fructose, galactose, starch, cellulose and its derivatives, polyols like mannitol, xylitol, sorbitol and their mixtures; maltodextrin; amino acids and their mixtures, and more particularly dextrose, lactose, mannitol, sorbitol or mixtures thereof, and the like, for example. The amino acids are, for example, involved in usual edulcorants. The filler can be used to obtain effective cohesion of the freeze dried particles.

Preferred fillers are sugar-type fillers, and a particularly preferred filler is dextrose.

The filler can be added to the solution or suspension of the gel in an amount of about 20% to 80% by weight of the obtained dried material. In other embodiments, the filler can be about 20% to 30%, 17% to 55%, or 17% to 30% by weight of the obtained dry material.

In the particular case of piroxicam complexed with BCDP, the preferred range of acid is of between 5 and 10% wt of the freeze dried material, binder can be in proportions of between 6 and 10% wt of the freeze-dried material, and the filler can be in proportions of between 20 and 30% wt of the freeze-dried material.

Advantageous upper ranges of preferred ingredients are described in the table below.

| Ingredient | weight % |
|---|---|
| β-Cyclodextrin | 47-59 |
| Piroxicam | 5-9 |
| Citric acid | 6-8 |
| Pectin | 6-8 |
| Dextrose | 22-27 |

As discussed in the Background section, while EP 84113923.1 discloses the use of BCDP, the formulations disclosed in said reference do not contain sufficient amounts of acid, binder, or filler to mask the unpleasant taste of this complex. More specifically, EP 84113923.1 fails to discuss taste masking at all, much less improving the palatability of an unpleasant tasting active substance through a non-lipidic combination of acid, binder and filler.

With respect to processes for making the compositions described herein, it should be noted that the disclosed methods are more efficient than those described in the prior art. The methods provided herein allow for all ingredients, in amounts sufficient to improve the palatability of an unpleasant tasting active substance, to be added prior to lyophilization. Thus the resulting palatable freeze-dried compositions contain the desired active substance and a sufficient amount of acid, binder and filler, to mask the unpleasant taste of the active substance.

Furthermore, the processes for making the compositions described herein do not require a coating step or the addition of a flavor enhancer in order to improve the palatability of an unpleasant tasting active substance. Thus the methods provided herein are more efficient in generating palatable pharmaceuticals than methods previously known in the art.

In certain embodiments, step a) of a process to make the oral compositions described herein can be carried out as disclosed in 84113923.1, and as it appears in the following examples.

Furthermore, step b) can be carried out by incorporating the acid, the binder and the filler into the aqueous or hydroalcoholic solution or suspension of the first step a). (Embodiment 1). This leads to an aqueous or hydroalcoholic gel.

However, according to another embodiment, step b) can be carried out by incorporating the aqueous or hydroalcoholic solution or suspension obtained in step a) into an aqueous gel by successively adding the acid, the binder, and then the filler to water. (Embodiment 2). This embodiment is preferred.

Preparation can also be carried out by incorporating a premix comprising the acid and the binder into the aqueous, hydroalcoholic solution, or suspension obtained in step a) and subsequently incorporating the filler. (Embodiment 3), or extemporaneously incorporating the filler (Embodiment 4).

Preparation can also be carried out by incorporating a premix comprising the acid, the binder and the filler into the aqueous, hydroalcoholic solution, or suspension of the active substance obtained in step a). (Embodiment 5). Preferably, step b) is made by incorporating the aqueous, hydroalcoholic solution, or suspension obtained in step a) into an aqueous gel by successively adding the acid, the binder and then the filler into water.

Turning now to the preferred embodiment 2, where the solution or suspension of the active substance is mixed with a gel, the gel can be prepared by successively incorporating the acid, the binder and the filler to water simultaneously or extemporaneously.

However in another embodiment (6), a premix of the acid and the binder is prepared and added to water, then the filler can be added.

In another embodiment (7), a premix of the acid, the binder and the filler is incorporated into water.

Any of the above embodiments lead to a gel which can then be directly freeze dried. Directly means here that the gel is not extruded.

According to the invention, the freeze dried material can be sieved, and then directly compressed to obtain tablets which are palatable. However, the obtained sieved particles can also be homogenized before any further processing step, such as a compression step, for example.

The mean diameter of the sieved particles depends on the mesh used. The mesh is preferably between about 200 µm to 5 mm. In the case of BCDP, the mesh as exemplified is of 1 mm. This is however not restrictive, and a mesh of about 0.5 to about 2 mm can be used.

The choice of the mesh depends on the active substance and of the selected acid, binder and filler, and the further process steps for producing the oral pharmaceutical compositions.

Namely in the formulation of the pharmaceutical composition, it is also possible, and it may be suitable, to add further excipients such as disintegration agents, effervescent agents, further flavoring agents and sweeteners, ligands, lubricants, solubilizers, colorings, diluents, wetting agents and mixtures thereof to the particles before or even after compression.

In particular, it can be useful to add preservatives and/or tensioactive substances to the gel before freeze-drying, and particularly in the solution or suspension of step a). Some of the active substances can be micronized and formulated with surfactants. For example, paracetamol can be solubilized with lauryl sulfate.

Furthermore, it may be suitable to add at least one additional excipient selected from the following list of excipients, tensioactives, lipids and preservatives, during the process of preparing the oral pharmaceutical compositions, whatever its form. A person skilled in the art will be able to formulate the oral composition based on the particles of the invention, and to choose the useful or necessary additional excipients from the lists below.

Excipients can include lactose, glycine, mannitol, xylitol, sorbitol, maltitol, glucose, saccharose, maltodextrin, dextrose, fructose, galactose, sodium phosphate, sodium chloride, aluminum silicates, glycine, L-alanine, L-aspartic acid, L-glutamic acid, L-hydroxyproline, L-isoleucine, L-leucine, L-phenylalanine, beta-cyclodextrin: dimethyl beta-cyclodextrin and hydroxypropyl beta-cyclodextrin, polysaccharides, carboxymethylcelluloses, reticulated sodium carboxymethylcellulose, precipitated silica, colloidal silica, starch, modified starch, directly compressed sugar, stearic acid, magnesium stearate, sodium stearate, sodium stearylfumarate, "macrogol 6000", leucine, benzoate sodium, polyethylene glycol, aspartame, saccharinate sodium, cyclamate, acesulfame K, alginates, dextrans, gelatin, soy protein, wheat and psyllium seed proteins, dextrin, arabic gum, guar, agar, xanthan, carrageenans, pectins, pectinates, cellulose, tragacanth, chitosan, sodium glycolate, curdlan gum, ethers of cellulose, carboxy methyl chitin, acrylic and methylacrylic polymers and copolymers, hydrolyzed dextrose, hydroxyethylcellulose, dried extract of corn syrup, caroub gum, konjac flour, rice flour, gluten of flour, potato protein, papain, ethylcellulose, hydroxyethylcellulose, hydroxypropylmethylcellulose, hydroxypropylcellulose, cellulose acetate phthalate, hydroxypropylcellulose phthalate, polymethacrylates, acetophtalate cellulose, hydroxyethylcellulose/hydroxypropylcellulose, polymethylsiloxannes, polyacrylamides, povidone and derivatives, vinyl polyacetate, polylactic acid, polyurethane, polypeptides, gelatin/arabic gum complex, and the like, for example.

Tensioactives can include polysorbates such as Tween 60 and Tween 80, sorbitum esters, polyethers of fatty glycerids, lecithin, sodium lauryl sulfate, sodium dioctysulfosuccinate, alkaline sulfate of lauryl acid (sodium laurylsulfate between 0.5 to 0.7%) monoethyl ether of diethyleneglycol and the like, for example.

Lipids can include beeswax, wax of carnouba, lavolin, stearic acid, oleic acid, sodium steryl fumarate, glycerin monostearate, glycerine palmitostearate, soya lecithin, yellowegg lecithin, phosphotidylcholine, phosphotidylethanolamina, phosphotidylinosine, phosphotidylserina, phosphatidic acid, cerebrosides, cholesterol, mineral oil, ricin oil, cotton seed oil, soya oil, peanut oil, copra oil, hydrogenated vegetable oil, hydrocarbons, fatty alcohol, and the like, for example.

Preservatives can include benzalkonium chloride, chlorhexidine, sodium or non sodium parabens, benzoic acid, sorbic acid, and the like, for example.

The following examples are only given with the purpose of better illustrating the invention, but in no way they must be construed as a limitation of its scope.

### EXAMPLE

### Steps for preparing palatable Formulations 1 to 14:

The constituents as listed in the following table were mixed and treated in line with the steps depicted at the last line of the table. These steps 1 to 10 were carried out according to the following steps (1) to (10). The bitterness, palatability and form of the compound after sieving were evaluated.

### (1). Preparation of the solution of complex for 200 units

34.24 g of β-cyclodextrin was added to 196 ml of water which had been heated to 75°C. The suspension was stirred during 30 minutes and subsequently 4 g of piroxicam and 4.18 g of aqueous 28% ammonium hydroxide was added. The liquid was stirred for 30 minutes at 75°C.

### (2). Preparation of the piroxicam solution (Formulation 10)

4 g of piroxicam was added to 196 ml of water which had been heated to 75°C. The suspension was stirred for 30 minutes at 75°C.

### (3). Preparation of the gel to be lyophilized

Citric acid (Formulation 9: fumaric acid) was added to the water which had been heated to 75°C. Apple pectin (Formulation 6: gelatin A) was then added, then the dextrose (Formulation 7: mannitol, Formulation 8: fructose, Formulation 13: dicalcic phosphate). The mixture was then stirred until dissolution and obtainment of an homogenous gel. The solution of complex (Formulation 10 solution of piroxicam) was incorporated into the gel at 75°C. The mixture was stirred and homogenized at 75°C until a homogeneous gel was obtained.

### (4). Preparation of the mixture to be lyophilized.

Aroma, then acid, then apple pectin, then dextrose were added to the solution of complex obtained in (1). The mixture was stirred at 75°C until complete dissolution and a homogeneous gel was obtained.

### (5). a) Preparation of a premix

Citric acid and apple pectin were mixed in a mixer (known under the commercial name Turbula T2C) until their mixture was homogeneous (10 minutes).

### b) Preparation of the mixture to be lyophilized.

The premix was added to the solution of complex as obtained in 1, then the dextrose was added.

The mixture was stirred at 75°C until complete dissolution and a homogeneous gel was obtained.

### (6). a) Preparation of a premix: idem (5).a)

b) Preparation of the mixture to be lyophilized: idem (5).b) except that the dextrose was added 30 minutes after the premix was added to water.

### (7). a) Preparation of a premix

Citric acid, then apple pectin, then dextrose were mixed in a mixer (known under the commercial name Turbula T2C) until the mixture was homogeneous (10 minutes).

### b) Preparation of the mixture to be lyophilized.

The premix was added to the solution of complex as obtained in 1.

The mixture was stirred at 75°C until complete dissolution and a homogeneous gel was obtained.

### (8). lyophilization and sieving

The mixture was then freeze-dried.

The obtained freeze dried material was sieved on a 1 mm mesh. It can be directly compressed.

### (9). a) Preparation of a premix

Citric acid, then apple pectin were mixed in a mixer (known under the commercial name Turbula T2C) until the mixture was homogeneous (10 minutes). The premix was added to water and dextrose was added immediately.

### b) Preparation of the mixture to be lyophilized.

The solution of complex as obtained in 1 was incorporated into the gel. The mixture was stirred at 75°C until complete dissolution and a homogeneous gel was obtained.

### (10). In another embodiment, citric acid, then apple pectin, then dextrose were mixed in a mixer as described above.

This premix was added to water and a homogeneous gel was obtained. The mixture to be lyophilized was prepared as above (9).b).

### Steps for preparing formulations 15 to 18:

The process was carried out with the steps as depicted at the last line of the table, except the omitted constituents.

In the above formulations, the palatability was evaluated by a panel of at least 3 persons. Bitterness was evaluated amongst no, +, ++ and +++. Palatability was evaluated amongst bad, neutral, pleasant. The results are provided in the table below.

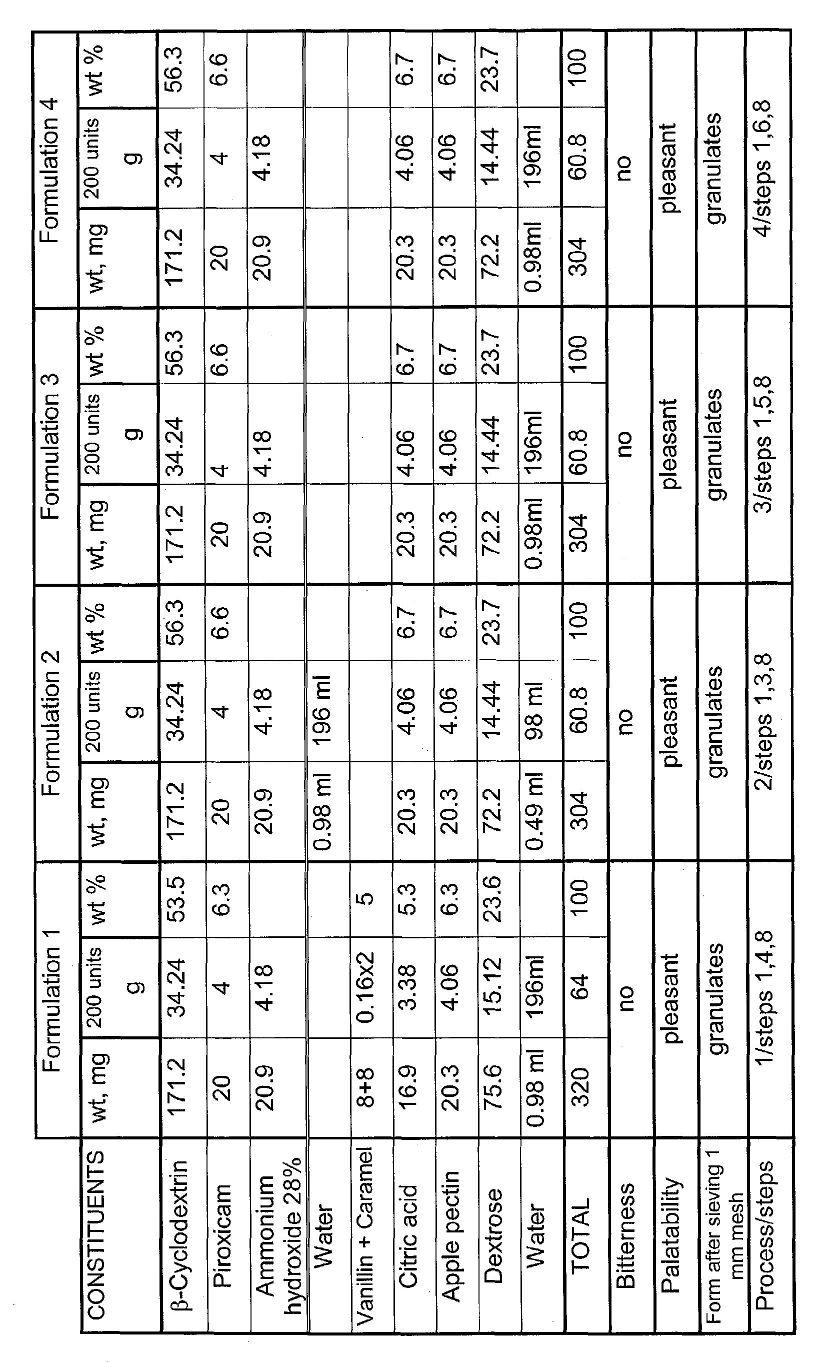

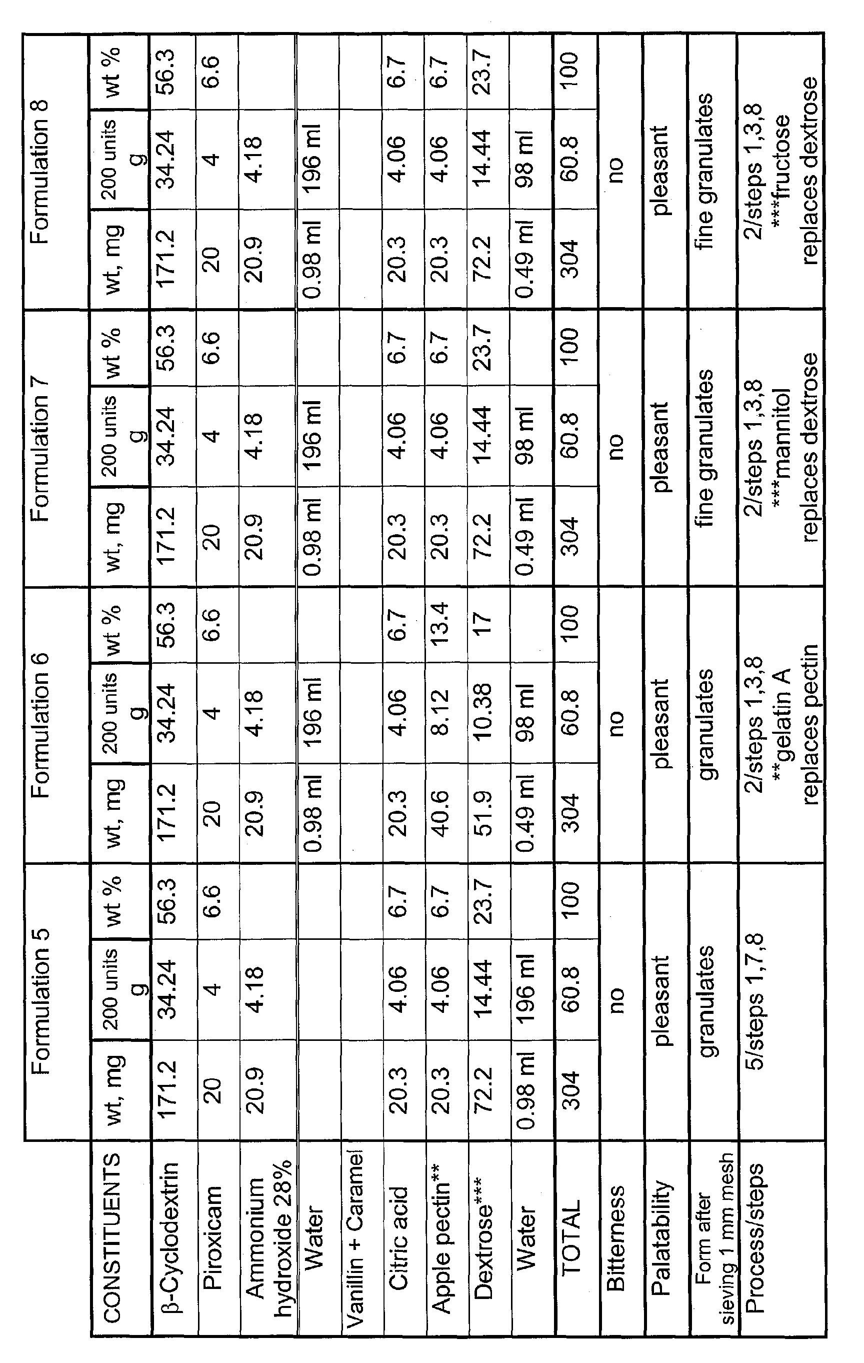

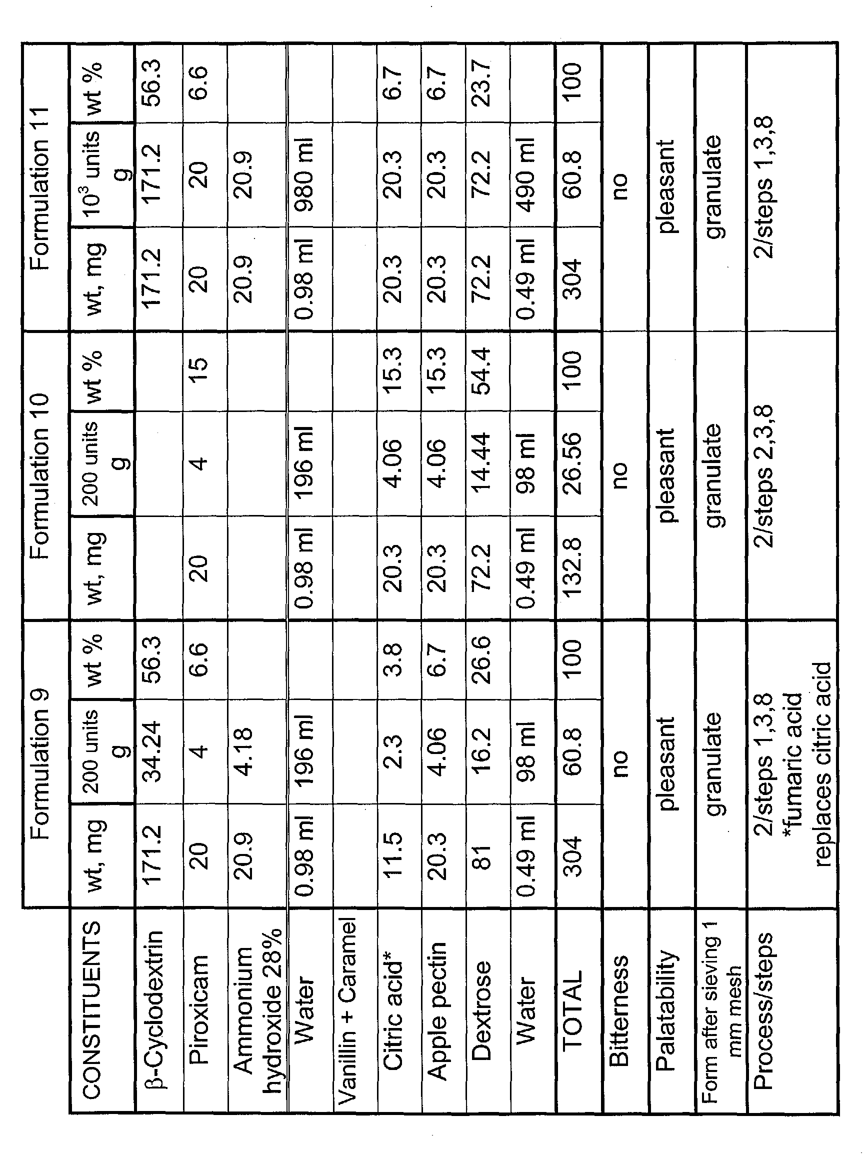

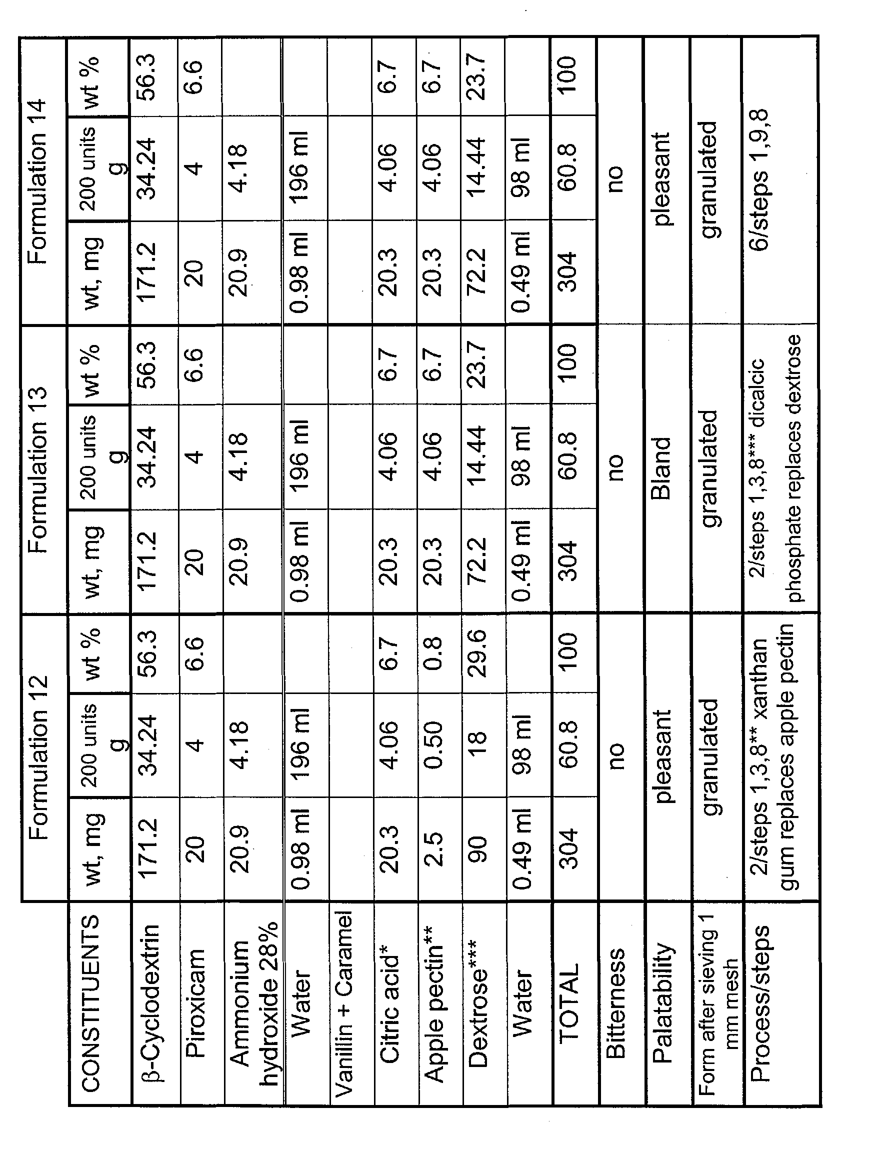

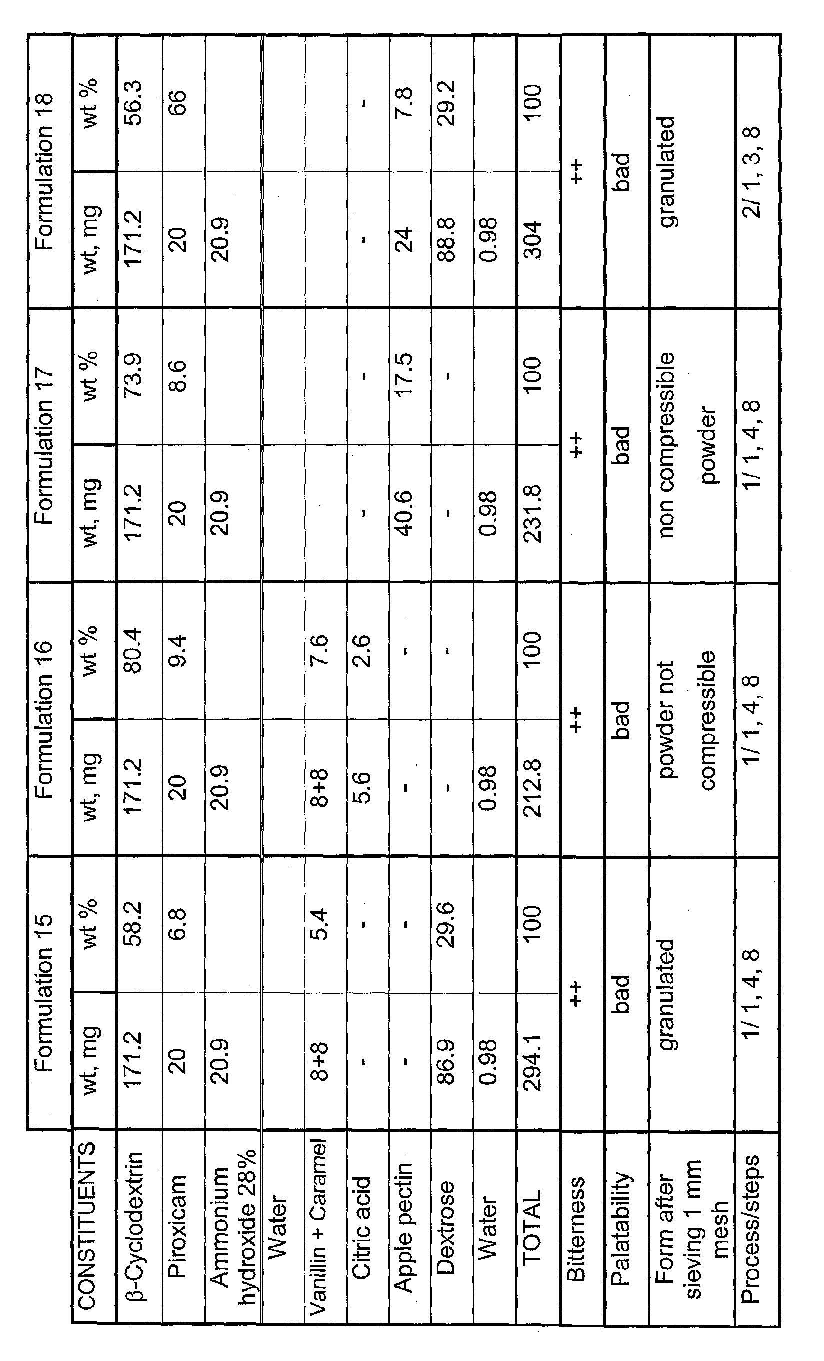

## Claims

**1.** A composition having improved palatability prepared by freeze drying a mixture comprising a) at least one pharmaceutically active substance having an unpleasant taste; and b) at least one non-lipidic taste masking association comprising: b1) at least one acid and b2) at least one non-lipidic binder in sufficient amount to improve the palatability of the active substance having an unpleasant taste, and c) at least one filler.

**2.** The composition according to claim 1, wherein said acid is selected from the group consisting of organic acids containing 2 to 8 carbon atoms.

**3.** The composition of claim 1 or 2, wherein said acid is citric acid or fumaric acid.

**4.** The composition of one of claims 1 to 3, wherein said non-lipidic binder is selected from the group consisting of pectin, gelatin, xanthan gum, guar gum, cellulose derivatives, soy derivatives, polysaccharides, pullolan, povidone and derivatives, polyethylene glycol, chitosan and its derivatives, fermentation obtained sugars like xanthan, alginates, and their mixtures.

**5.** The composition of one of claims 1 to 4, wherein said binder is selected from the group of pectin, gelatin, xanthan gum, and apple pectin, and their mixtures.

**6.** The composition of one of claims 1 to 5, wherein the filler is selected from the group consisting of dextrose, lactose, saccharose, fructose, galactose, starch, cellulose and its derivatives, polyols like mannitol, xylitol, sorbitol, maltodextrin, amino acids, and their mixtures.

**7.** The composition of one of claims 1 to 6, wherein the filler is dextrose.

**8.** The composition of one of claims 1 to 7, wherein the pharmaceutically active substance is selected from the group consisting of non steroidal anti-inflammatory drugs, antacids, analgesics, antalgics, sedatives, antipyretics, antispasmodics, coronary vasodilatators, peripheral vasodilatators, cerebral vasodilatarors, anti-injectives, antibiotics, antiparasitics, antineoplasics, anxiolytics, tranquilizers, CNS stimulating agents, antihistaminics, laxatives, drugs for digestive motricity, anticoagulant, drug for use in thyroid dysfunction, nutriments, nutritional adjuvants, immuno-depressives, cholesterol-lowering agents, hormones, sex hormones, enzymes, antianginal drugs, anti-migraine drugs, myorelaxants, hypoglycemics, anorexiants, expectorants, antitussives, decongestants, antinauseants, hematopoietic drugs, uricosurics, plant extracts, radio contrast media, anti-anginals, anti-anxiety drugs, anti-arrhythmics, anti-bacterials, anti-diarrhoeals, anti-depressants, antiepileptics, anti-fungals, anti-hypertensives, anti-inflammatory agents, anti-virals, cardiac agents, contraceptives, cough suppressants, cytotoxics, decongestants, diuretics, drugs for genito-urinary disorders, drugs for asthma disorders, drugs for use in Parkinson's Disease, and related disorders, drugs for use in rheumatic disorders, hypnotics, minerals, vitamins, lipid lowering drugs and the like.

**9.** The composition of one of claims 1 to 8, wherein the pharmaceutically active substance is selected from the group consisting of piroxicam, piroxicam-β-(cyclodextrine), ambroxol hydrochloride, paracetamol, domperidone, nimesulide, ibuprofen, morniflumate, apomorphine, and dihydroergocryptine.

**10.** The composition of one of claims 1 to 9 and 11, wherein the pharmaceutically active substance is non-complexed piroxicam or paracetamol.

**11.** The composition according to one of claims 1 to 9, wherein the pharmaceutically active substance is complexed with a cyclodextrin.

**12.** The composition according to one of claims 1 to 9 and 11, wherein the pharmaceutically active substance is piroxicam-β-(cyclodextrine).

**13.** The composition according to one of claims 1 to 12, comprising at least 5% by weight of the acid.

**14.** The composition according to one of claims 1 to 13, comprising at least 6% by weight of the binder.

**15.** The composition according to one of claims 1 to 14, comprising at least 20% by weight of the filler.

**16.** The composition according to one of claims 1 to 15, wherein the active substance is complexed piroxicam, and the weight ratio between the acid and binder is equal to or less than 1.

**17.** The composition according to one of claims 1 to 16, wherein the active substance is complexed piroxicam, and the acid is citric acid and the binder is apple pectin.

**18.** The composition according to one of claims 1 to 17, wherein the active substance is BCDP, and the percentage of acid, based on total weight, is between 5 and 10%, the percentage of the binder, based on total weight, is between 6 and 10% and the percentage of the filler, based on total weight, is between 20 and 30%.

**19.** The composition according to one of claims 1 to 18, wherein the active substance is BCDP, and the percentage of citric acid, based on total weight, is between 5 and 10%, the percentage of the pectin, based on total weight, is between 6 and 10% and the percentage of the dextrose, based on total weight, is between 20 and 30%.

**20.** The composition according to one of claims 1 to 19, wherein the composition is a freeze-dried aqueous or hydroalcoholic gel.

**21.** The composition according to one of claims 16 to 20, wherein the composition is a freeze-dried aqueous or hydroalcoholic gel is a free-flowing compressible powder.

**22.** The composition according to one of claims 1 to 21, wherein the composition is an oral pharmaceutical.

**23.** Sieved freeze dried particles comprising a) at least a pharmaceutically active substance which has an unacceptable taste; b) at least a non-lipidic taste masking association comprising: b1) at least an acid and b2) at least a non-lipidic binder; and c) at least a filler.

**24.** Free-flowing compressible powder-like freeze-dried particles comprising a) at least a pharmaceutically active substance which has an unacceptable taste; b) at least a non-lipidic taste masking association comprising: b1) at least an acid and b2) at least a non-lipidic binder; and c) at least a filler.

**25.** A process for the preparation of freeze-dried particles comprising a pharmaceutically active substance having an unpleasant taste, and a non-lipidic taste masking association comprising an acid and a binder, and a filler, said process comprising the following steps:
a) preparing an aqueous or hydroalcoholic solution or suspension of the active substance having an unpleasant taste;
b) preparing an aqueous or hydroalcoholic gel comprising said aqueous or hydroalcoholic solution or suspension and a non-lipidic binder, an acid, and a filler;
c) homogenizing the aqueous or hydroalcoholic gel obtained in step b);
d) freeze-drying said homogenized gel; and
e) recovering the freeze-dried material.

**26.** The process of claim 25, wherein the freeze-dried composition resulting from step d) is sieved and recovered.

**27.** The process of claim 25 or 26, wherein the acid, the binder, and the filler provided in step b) are successively added, either simultaneously or extemporaneously, to the solution or suspension.

**28.** The process of one of claims 25 to 27, wherein the at least one pharmaceutically active substance is BCDP.

**29.** The process of one of claims 25 to 28, further comprising sieving the freeze-dried material obtained from step d) through a mesh of between about 200 µm to 5 mm.

**30.** The process of one of claims 25 to 29, further comprising preparing a pharmaceutical oral composition comprising the recovered freeze-dried material from step e).

**31.** A pharmaceutical composition having improved palatability, comprising:
a) at least one active substance having an unpleasant taste;
b1) at least one a non-lipidic binder and
b2) at least an acid; and
c) at least a filler
wherein said composition results from lyophilization of a homogeneous liquid or gel.

**32.** Pharmaceutical oral composition comprising freeze dried particles of claims 1 to 24 or obtained by the process of claims 25 to 29.

**33.** The pharmaceutical composition of claim 31 or 32, in the form of a particulate material.

**34.** The pharmaceutical composition of claim 31 or 32, further comprising acceptable vehicle for a sachet form.

**35.** The pharmaceutical composition of one of claims 31 or 32, formed into a tablet.

**36.** A method for making a pharmaceutical material having improved palatability, comprising at least:
preparing a homogeneous liquid or gel comprising
a) at least one active substance having an unpleasant taste;
b1) at least one a non-lipidic binder and
b2) at least an acid; and
c) at least a filler
wherein said composition results from lyophilization of a homogeneous liquid or gel and
lyophilizing said liquid or gel.

**39.** The method of claim 38 further comprising seeving and/or compressing the freeze-dried liquid or gel.

**40.** The method of claim 38 or 39, further comprising formulating an oral pharmaceutical composition from the lyophilized gel or liquid.
